# EUROPÄISCHE PATENTANMELDUNG

(11) **EP 1 747 818 A2**
(43) Veröffentlichungstag der Anmeldung: **31.01.2007**
(21) Anmeldenummer: 06014899.6
(22) Anmeldetag: 18.07.2006
(51) Int. Cl.: B06B 1/02

(54) **System und Verfahren zur Erzeugung von Ultraschallwellen**

(30) Priorität: 27.07.2005 EP 05016273
(71) Anmelder: Wellcomet GmbH, 76344 Eggenstein-Leopoldshafen (DE)
(72) Erfinder: Kruglikov, Ilja, Dr., 76351 Linkenheim Hochstetten (DE)
(74) Vertreter: Brommer, Hans Joachim

(57) **Zusammenfassung**

System zur Erzeugung von Ultraschallwellen, insbesondere zur kosmetischen und therapeutischen Behandlung mit einem elektrisch angesteuerten Ultraschallkopf, der Ultraschallwellen mit zumindest zwei unterschiedlichen Frequenzen *f₁, f₂* erzeugt, wobei eine Steuereinheit permanent oder zumindest während vorgegebener Zeitspannen zwischen den zumindest zwei vorgegebenen Frequenzwerten *f₁, f₂* umschaltet. Wesentlich dabei ist, dass das Umschalten mit einer Wechselfrequenz im Bereich von 10 Hz bis 10 000 Hz, insbesondere im Bereich von 50 Hz bis 5 000 Hz, vorzugsweise 100 Hz bis 2000 Hz erfolgt.

## Beschreibung

Die Erfindung betrifft ein System zur Erzeugung von Ultraschallwellen gemäß dem Oberbegriff des Anspruchs 1, sowie ein Verfahren zur Erzeugung von Ultraschallwellen gemäß dem Oberbegriff des Anspruchs 14.

Ultraschallwellen werden zur Beaufschlagung von biologischem Gewebe für medizinische, therapeutische oder kosmetische Anwendungen, sowie für Forschungszwecke benutzt. Üblicherweise werden mit dem Begriff Ultraschallwellen Schwingungen mit einer Frequenz größer 20 kHz bezeichnet. Häufig ist hierbei die Verwendung von niederfrequenten Ultraschallfrequenzen im Bereich von 20 kHz bis 100 kHz und die Verwendung von hochfrequenten Ultraschallfrequenzen über 0,8 MHz.

Systeme zur Erzeugung von Ultraschallwellen für die Behandlung von biologischem Gewebe weisen üblicherweise einen Signalgenerator auf, welcher zur Erzeugung von periodischen elektrischen Signalen dient. Ferner umfassen typische Systeme einen Ultraschallkopf, welcher mittels einer elektrischen Leitung mit einem Signalausgang des Signalgenerators verbunden ist. Der Ultraschallkopf besitzt einen Bereich zur Auflage auf das biologische Gewebe, wobei im Ultraschallkopf in der Nähe dieses Auflagebereiches die vom Signalgenerator erzeugten periodischen elektrischen Signale in Ultraschallwellen umgewandelt werden.

Typischerweise.ist ein Ultraschallkopf auf die Umwandlung von elektrischen Signalen mit einer festen Frequenz ausgelegt. Es sind jedoch auch Ultraschallköpfe bekannt, welche elektrische Signale mit unterschiedlichen Frequenzen in entsprechende Ultraschallwellen umwandeln können:

Die US 5,460,595 beschreibt ein System zur Erzeugung von Ultraschallwellen, bei dem der Signalgenerator und der Ultraschallkopf derart ausgelegt sind, dass Ultraschallwellen mit drei verschiedenen Frequenzen erzeugt werden können, ohne dass der Ultraschallkopf ausgetauscht werden müsste.

Dieses System stellt dem Anwender drei verschiedene Ultraschallfrequenzen zur Verfügung, um drei verschiedene Eindringtiefen in das biologische Gewebe zu bewirken.

Wie tief und mit welcher Intensität der Ultraschall in den Körper eindringt, hängt von der Frequenz der Ultraschallwelle ab. Ein Maß für die Eindringtiefe ist die so genannte Halbwertstiefe, das heißt die Strecke, nach der sich die Schallintensität in dem biologischen Gewebe auf 50 % durch Absorption vermindert hat. Je geringer die Frequenz, desto höher die Eindringtiefe. Für typisches biologisches Gewebe eines Menschen beträgt die Halbwertstiefe einer Ultraschallwelle mit Frequenz 3 MHz etwa 1 cm und für eine Ultraschallwelle mit der Frequenz 1 MHz etwa 3 cm.

Die Halbwertstiefe ist unabhängig von der eingestrahlten Intensität, wohingegen die bis zu einer gewissen Tiefe in dem biologischen Gewebe absorbierte Energie sowohl von der Frequenz als auch von der Intensität der Ultraschallstrahlung abhängt.

Mit dem bekannten System lässt sich somit die Halbwertstiefe mittels der benutzten Ultraschallfrequenz und die bis zu einer bestimmten Tiefe absorbierte Energie mittels der gewählten Intensität variieren. Wünschenswert wäre es jedoch, bei der Ultraschallbehandlung die Massagewirkung, das heißt die Relativbewegung einzelner Teile des behandelten biologischen Gewebes stärker beeinflussen zu können.

Systeme mit den Merkmalen des Oberbegriffes des Anspruches 1 und 14 sind durch die US 6148225, EP 1 262 160 und US 5178134 bekannt geworden. Dort wird jeweils die Behandlungsfrequenz ständig geändert.

Der Erfindung liegt die Aufgabe zugrunde, ein System und ein Verfahren zur Erzeugung von Ultraschallwellen zu schaffen, welches die Möglichkeiten der Beeinflussung von biologischem Gewebe durch Ultraschallwellen erweitert, insbesondere neue Massagewirkungen ermöglicht.

Diese Aufgabe wird erfindungsgemäß dadurch gelöst, dass das Umschalten zwischen den zumindest zwei unterschiedlichen Frequenzen mit einer Wechselfrequenz im Bereich 10 Hz bis 10 000 Hz, insbesondere im Bereich von 50 Hz bis 5 000 Hz, vorzugsweise von 100 Hz bis 2 000 Hz erfolgt. Hierdurch ergibt sich überraschenderweise eine gegenüber den bekannten Systemen wesentlich intensivere Massagewirkung im Gewebe.

Dabei geht die Erfindung von folgender Erkenntnis aus:

Die Ultraschallbeaufschlagung führt in dem biologischen Gewebe abwechselnd zu Bereichen mit Überdruck und Bereichen mit Unterdruck, wobei der Druckwert, das heißt insbesondere die Differenz des Drucks zwischen den Bereichen mit Überdruck und den Bereichen mit Unterdruck frequenzunabhängig ist.

Frequenzabhängig ist jedoch die Entfernung der Bereiche mit Überdruck und der Bereiche mit Unterdruck. Zwei Bereiche mit Überdruck sind in etwa um eine Strecke voneinander entfernt, welche der Wellenlänge der verwendeten Ultraschallstrahlung entspricht. Etwa mittig zwischen diesen Bereichen mit Überdruck, das heißt in etwa eine halbe Wellenlänge entfernt, befindet sich ein Bereich mit Unterdruck. Je höher die Frequenz, desto geringer die Wellenlänge und damit der Abstand zwischen Bereichen mit Über- und Unterdruck. Auch wenn sich die Höhe des Drucks mit der Frequenz der verwendeten Ultraschallstrahlung nicht ändert, so ändert sich jedoch der Abstand zwischen Bereichen mit Über- und Unterdruck und damit der Druckgradient im Gewebe.

Nach Untersuchungen der Anmelderin ist die Geschwindigkeit, mit der zwischen den mindestens zwei Ultraschall-Frequenzen gewechselt wird, also die Umschaltfrequenz, ganz wesentlich für den therapeutischen Effekt der Behandlung. Die Umschaltfrequenz gibt an, wie oft pro Sekunde ein Frequenzwechsel stattfindet, d. h. von der aktuellen Ultraschallfrequenz zu der nächsten Ultraschall-Frequenz übergegangen wird.

Dabei hat sich herausgestellt, dass die erfindungsgemäßen Umschaltfrequenzen in einer optimalen Relation zur Eigenelastizität der Gewebezellen, insbesondere der Zellmembranen stehen. So wurde durch Anwendung der genannten Umschaltfrequenzen eine bedeutend intensivere Einwirkung auf die Zelle registriert mit einer Steigerung der Durchlässigkeit der Zellmembranen bis auf 200 %. Diese Verbesserung der Zell-Diffusion führt zu einer deutlichen Straffung des Gewebes, was insbesondere bei Cellulites von Vorteil ist, aber auch zur Behandlung von Arthrose und Arthritis eingesetzt werden kann.

Daneben ist auch die Wahl der Ultraschall-Frequenzen von Bedeutung. Vorzugsweise wird mit Frequenzen von 0,7 MHz bis etwa 10 MHz gearbeitet, wobei die geringeren Frequenzen für eine hohe Eindringtiefe von einigen Zentimetern in das Gewebe, die hohen Frequenzen für eine eher oberflächliche Gewebebehandlung mit einer Eindringtiefe von weniger als 1 Zentimeter empfehlenswert sind.

Wesentlich ist schließlich noch, dass die Frequenzwerte, zwischen denen hin und her geschaltet wird, relativ weit auseinander liegen sollten, nämlich im Verhältnis 1:2 bis 1:10, insbesondere 1:2,5 bis 1:5. Es hat sich gezeigt, dass bei derartigen Frequenzabständen die Zellen intensiver beaufschlagt werden als bei Frequenzabstähden, die außerhalb dieses Bereiches liegen.

Grundsätzlich stehen dem Fachmann für die Erzeugung der Ultraschallwellen wie auch für die Umschaltung zwischen den unterschiedlichen Frequenzen verschiedene Möglichkeiten zur Verfügung. Besonders zweckmäßig ist es, wenn mit einem Signalgenerator gearbeitet wird, der zumindest zwei Frequenzen erzeugt, die in Ultraschallwellen mit entsprechenden Frequenzen umgewandelt werden, und dass die Steuereinheit die Umschaltung zwischen den Frequenzen bewirkt. In erster Linie ist hierbei an eine Umschaltung zwischen den Frequenzen der elektrischen Signale des Signalgenerators gedacht. Es liegt aber auch im Rahmen der Erfindung, stattdessen oder zusätzlich zwischen den Frequenzen der Ultraschallwellen umzuschalten.

Bei der erstgenannten Alternative kann der Signalgenerator einen Oszillator und eine Frequenz-Modulationseinheit aufweisen, die aus der Oszillatorfrequenz zumindest zwei verschiedene Frequenzen erzeugt. In diesem Fall braucht der Oszillator nur eine Frequenz erzeugen, aus der die Modulationseinheit dann die gewünschten unterschiedlichen Frequenzen ableitet.

In einer vorzugsweisen Ausführungsform weist der Signalgenerator einen Oszillator, einen Frequenzcontroller und eine Verstärkungseinheit auf. Der Oszillator erzeugt die Grundschwingung, wobei die Frequenz, mit der der Oszillator schwingt, über den mit dem Oszillator verbundenen Frequenzcontroller reguliert wird. Der Ausgang des Oszillators ist mit dem Eingang der Verstärkungseinheit verbunden, so dass am Ausgang der Verstärkungseinheit das Schwingungssignal des Oszillators in verstärkter Form vorliegt. Der Verstärkungsfaktor kann an der Verstärkungseinheit vorgegeben werden. Der Ausgang der Verstärkungseinheit ist mit dem Signalausgang des Signalgenerators verbunden, sodass das verstärkte Oszillatorsignal an den Ultraschallkopf weitergeleitet wird.

In einer weiteren vorzugsweisen Ausführungsform ist die Steuereinheit als Mikrocontroller ausgeführt, das heißt als Kontrolleinheit, welche eine elektronische Steuerung umfasst. Der Mikrocontroller kann beispielsweise durch einen Computer realisiert sein. Über einen Steuerausgang ist der Mikrocontroller mit dem Eingang des Frequenzcontrollers verbunden, sodass der Mikrocontroller letztendlich die Frequenz vorgibt, welche am Signalausgang des Signalgenerators anliegt.

Weiterhin ist es vorteilhaft, wenn die Steuereinheit zusätzlich die Intensität der Ultraschallwelle steuert. Hierzu kann die Steuereinheit zum einen mit einem Steuereingang der Verstärkungseinheit verbunden sein und zum anderen über einen Signaleingang mit einem Signalausgang der Verstärkungseinheit verbunden sein. Über den Signaleingang der Steuereinheit kann somit die tatsächliche Intensität des am Signalausgang des Signalgenerators anliegenden Signals überprüft werden und entsprechend kann der Verstärkungsfaktor korrigiert und entsprechend an den Steuereingang der Verstärkungseinheit weitergeleitet werden, sodass am Signalausgang ein Signal mit einer vorgegebenen Intensität anliegt.

Darüber hinaus ist es vorteilhaft, wenn das System zur Erzeugung von Ultraschallwellen mindestens zwei Ultraschallköpfe mit unterschiedlicher Größe aufweist. Ultraschallköpfe mit unterschiedlicher Größe haben eine unterschiedlich große Auflagefläche zur Auflage auf das zu bestrahlende biologische Gewebe. Je nach Form des Gewebes und abhängig von der gewünschten zu bestrahlenden Fläche kann so ein passender Ultraschallkopf ausgewählt werden. Hierzu ist der Signalausgang des Signalgenerators derart ausgeführt, dass wahlweise ein oder die mindestens zwei Ultraschallköpfe gleichzeitig mit dem Signalausgang verbunden werden können. Dadurch kann entweder ein passender Ultraschallkopf zur Verwendung ausgewählt werden oder es können mehrere Ultraschallköpfe angeschlossen werden, sodass gleichzeitig mehrere Stellen des biologischen Gewebes mit Ultraschallstrahlung beaufschlagt werden können.

In einer weiteren vorteilhaften Ausführungsform weist das System mindestens zwei Ultraschallköpfe auf, welche mit dem Signalausgang des Signalgenerators verbunden werden können, wobei der Signalausgang eine Signalsteuereinheit umfasst, welche automatisch das Signal an jeweils einen der angeschlossenen Ultraschallköpfe weiterleitet. Die Signalsteuereinheit weist einen Speicher zum Abspeichern von Behandlungsprogrammen auf, in denen die Behandlungsdauer und eventuell weitere Parameter wie Stärke und Frequenz der Ultraschallstrahlung oder Umschaltfrequenz für einen bestimmten Ultraschallkopf vorgegeben sind. Die Signalsteuereinheit ist hierfür mit einer Steuereinheit der Ultraschallköpfe verbunden, zur Steuerung der genannten weiteren Parameter. Nach Ablauf der vorgegebenen Behandlungsdauer schaltet die Signalsteuerung automatisch auf den nächsten im Programm angegebenen Ultraschallkopf um und gibt dies durch ein optisches und/oder akustisches Signal an. Weiterhin übermittelt die Signalsteuerung die weiteren Parameter an die Steuereinheit.

Die Behandlung kann daraufhin mit dem nun aktiven Ultraschallkopf mit den vorgegebenen weiteren Parametern fortgesetzt werden.

Um die Kombination einer Beschallung des biologischen Gewebes mit Ultraschall mit einer Reizstrombehandlung zu ermöglichen, weist das System in einer weiteren vorteilhaften Ausführungsform einen Reizstromgenerator auf, welcher einen Reizstrom generiert und an einem Reizstromausgang ausgibt. Der Reizstromausgang ist mit einer Kontakteinheit, welche beispielsweise als an sich bekannte Elektrode ausgeführt sein kann, verbunden, sodass über die Kontakteinheit der Reizstrom auf das biologische Gewebe übertragen werden kann. Die Kontakteinheit ist in den Ultraschallkopf integriert, sodass durch Auflegen des Ultraschallkopfes auf das biologische Gewebe sowohl der Reizstrom als auch die Ultraschallstrahlung in das Gewebe eindringen können.

Ebenso ist eine Kombination des erfindungsgemäßen Systems zur Erzeugung von Ultraschallwellen mit einer Vakuumeinheit vorteilhaft. Hierzu weist das erfindungsgemäße System eine Vakuumeinheit mit einem Vakuumanschluss auf, wobei die Vakuumeinheit derart an dem Ultraschallkopf angeordnet ist, dass bei Anlegen des Ultraschallkopfes an das biologische Gewebe ein Unterdruck erzeugt werden kann, welcher das biologische Gewebe an den Ultraschallkopf andrückt. Die Vakuumeinheit kann beispielsweise als Vakuumglocke ausgebildet sein. Diese bildet mit dem Ultraschallkopf eine bauliche Einheit, so dass durch Auflegen des Ultraschallkopfes auf das biologische Gewebe automatisch eine annähernd fluiddichte Verbindung zwischen biologischem Gewebe und Vakuumglocke hergestellt werden kann.

Ebenso ist die Kombination des erfindungsgemäßen Systems zur Erzeugung von Ultraschallwellen mit einer Infraroteinheit zur Wärmebehandlung des biologischen Gewebes vorteilhaft. Hierzu weist das erfindungsgemäße System eine Infraroteinheit zur Erzeugung von Infrarotstrahlung auf, welche eine Infrarotstrahlungsquelle, wie beispielsweise eine Glühröhre, und eine Energieversorgung für Infrarotstrahlungsquelle umfasst. Die Infrarotstrahlungsquelle ist in den Ultraschallkopf integriert und derart ausgerichtet, dass bei Auflegen des Ultraschallkopfs auf das biologische Gewebe die Infrarotstrahlung der Infrarotstrahlungsquelle auf die Oberfläche des biologischen Gewebes einwirkt.

Besonders zweckmäßig ist die Kombination des beschriebenen Systems mit einer Vorrichtung zur Erzeugung von Hochfrequenzströmen, die mit einer Frequenz zwischen 300 kHz bis 10 MHz über zumindest eine nach außen laufende Elektrode auf das Gewebe übertragen werden. Dies kann gleichzeitig oder zeitlich versetzt zur Ultraschallbehandlung erfolgen.

Eine besonders vorteilhafte Anwendung der beschriebenen Ultraschallbehandlung ergibt sich bei Kombination mit der kosmetischen Fettabsaugung. Ursächlich hierfür ist die durch die erfindungsgemäße Ultraschallbehandlung signifikant verbesserte Durchlässigkeit der Zellwände. Dadurch kann von der gleichen Absaugstelle Fett aus wesentlich größerer Entfernung als bisher abgesaugt werden. Gleichzeitig vergleichmäßigt die bessere Durchlässigkeit der Zellen die lokale Fettkonzentration. Einbeulungen oder Wellenbildung an den behandelten Körperpartien werden dadurch ausgeschlossen.

Dabei erfolgt die Ultraschall-Behandlung zweckmäßig einmal direkt vor dem Fettabsaugen und insgesamt drei- bis viermal nach der Fettabsaugung, vorzugsweise täglich oder jeden 2. Tag. Die Behandlungszeit liegt vorzugsweise jeweils zwischen 30 und 60 min.

Weitere Einzelheiten und Merkmale der Erfindung ergeben sich aus den übrigen Ansprüchen und der nachfolgenden Beschreibung eines Ausführungsbeispieles sowie aus der Zeichnung; dabei zeigt
- Figur 1: eine perspektivische Ansicht eines erfindungsgemäßen Ultraschallkopfes und
- Figur 2: ein Blockschaltbild eines erfindungsgemäßen Systems zur Erzeugung von Ultraschallwellen.

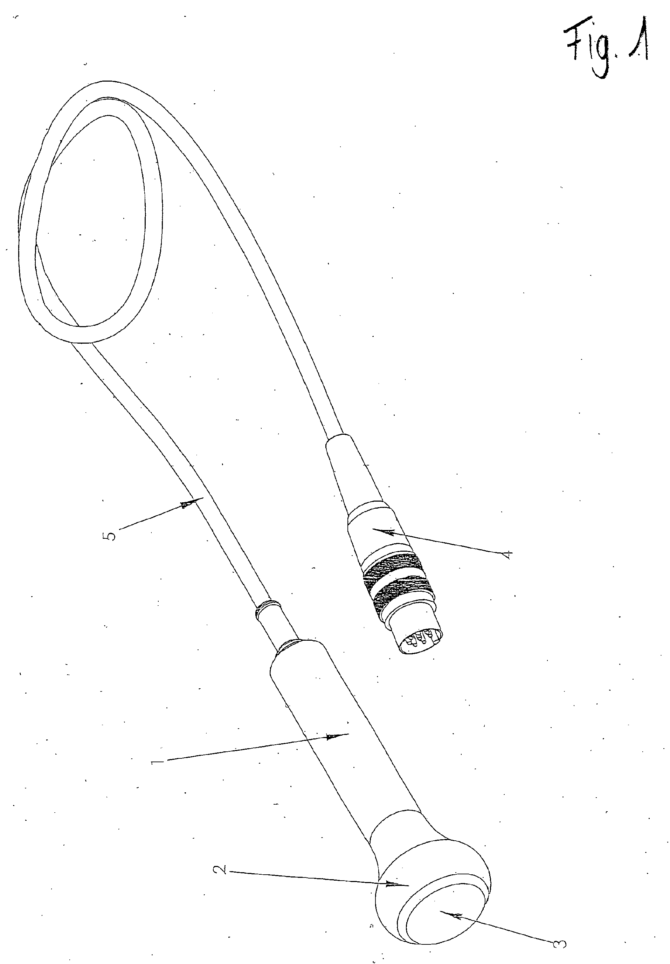

Der in Figur 1 dargestellte Ultraschallkopf umfasst ein Gehäuse 1, weiches an dem vorderen Ende ein Kopfteil 2 aufweist, an dem ein Protektor 3 angeordnet ist. An dem Protektor 3 ist eine Auflagefläche ausgebildet, zum Anlegen an das biologische Gewebe. Auf der im Kopfteil 2 liegenden Seite des Protektors 3 ist an dem Protektor ein Piezoelement angebracht, welches die Ultraschallschwingungen erzeugt und an den Protektor 3 weitergibt. In dem Gehäuse 1 ist ferner eine entsprechende Ansteuerelektronik für das Piezoelement enthalten, welche über eine Anschlussleitung 5 und einen Stecker 4 mit einem (nicht dargestellten) Signalgenerator verbunden werden kann. Die in dem Gehäuse 1 enthaltene Ansteuerelektronik wandelt in Verbindung mit dem Piezoelement elektrische Signale, welche über das Kabel 5 übertragen werden, in Ultraschallwellen um, sodass durch Auflegen des Protektors 3 auf biologisches Gewebe dieses mit Ultraschallwellen beaufschlagt werden kann.

Das Gehäuse 1 umfasst ferner eine Kopplungskontrolle 2. Die Kopplungskontrolle registriert, ob der Protektor 3 mit Druck beaufschlagt ist, das heißt ob der Protektor 3 an biologischem Gewebe anliegt. Ist dies der Fall, so gibt die Kopplungskontrolle 2 ein entsprechendes Signal über das Kabel 5 und den Stecker 4 an den (nicht dargestellten) Mikrocontroller des erfindungsgemäßen Systems zur Erzeugung von Ultraschallstrahlung.

In dem Stecker 4 befindet sich ferner ein Speicherbaustein, auf dem Kalibrierungsdaten des Ultraschallkopfes, insbesondere des Piezoelementes gespeichert sind. Der Speicherbaustein kann über in dem Stecker 4 angebrachte Kontakte mit dem Mikrocontroller verbunden werden, sodass der Mikrocontroller die gespeicherten Daten auslesen und abhängig von den Kalibrierungsdaten die Intensität der am Signalausgang des Signalgenerators anliegenden Signale derart wählen, dass eine Ultraschallstrahlung mit einer vorgegebenen Intensität durch das Piezoelement erzeugt wird.

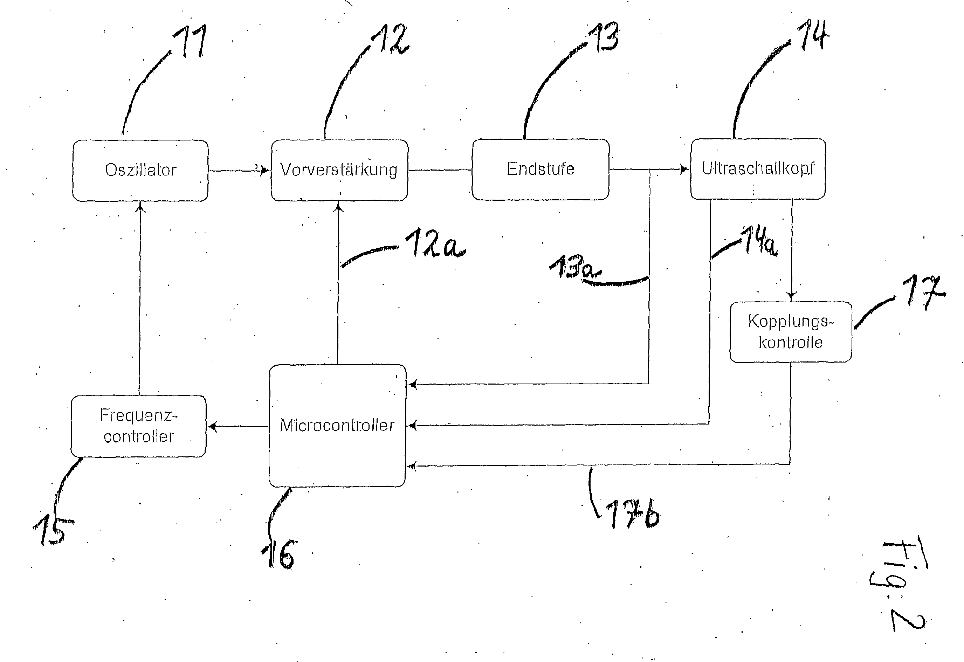

In Figur 2 ist ein Blockschaltbild des erfindungsgemäßen Systems zur Erzeugung von Ultraschallstrahlung dargestellt. Ein Oszillator 11 erzeugt periodische elektrische Signale, die anschließend in einer Verstärkungseinheit verstärkt werden. Diese Verstärkungseinheit besteht aus einer Vorverstärkung 12 und einer Endstufe 13. Das von der Endstufe ausgegebene Signal wird durch den in Figur 1 dargestellten Stecker 4 und das Kabel 5 an den Ultraschallkopf weitergeleitet, so dass im Ultraschallkopf 14 das elektrische Signal in Ultraschallwellen umgewandelt werden kann.

Die Frequenz, mit der der Oszillator 11 schwingt, wird durch einen Frequenzcontroller 15 reguliert. Dieser wird wiederum von einer Steuereinheit 16, welche als Microcontroller ausgeführt ist gesteuert, welcher die aktuell zu erzeugende Frequenz vorgibt. Für den Betrieb sind in dem Mikrocontroller folgende Größen vorgegeben:
- Die gewünschte Intensität der Ultraschallstrahlung,
- die Ultraschallfrequenzen, zwischen denen gewechselt werden soll,
- die Reihenfolge, in der zwischen den vorgegebenen Ultraschallfrequenzen gewechselt werden soll und
- die Umschaltfrequenz, mit der die Ultraschallfrequenzen gemäß der vorgegebenen Reihenfolge gewechselt werden.

Sind beispielsweise drei Ultraschallfrequenzen 1 MHz, 3 MHz und 10 MHz, eine Wechselreihenfolge (1 MHz, 3 MHz, 10 MHz, 3 MHz, 1 MHz, etc.), eine Intensität 1 W/cm², und eine Wechselfrequenz von 100 Hz vorgegeben, so gibt der Mikrocontroller zunächst für 0,01 sec das Signal an den Frequenzcontroller 15 zur Erzeugung einer Frequenz mit 1 MHz, danach für 0,01 sec das Signal zur Erzeugung einer Frequenz mit 3 MHz, dann für 0,01 sec das Signal zur Erzeugung einer Frequenz mit 10 MHz und wiederholt diesen Zyklus fortlaufend aufwärts und abwärts.

Demgemäß wird ein entsprechendes periodisches Signal mit der gewünschten Frequenz erzeugt und liegt am Ausgang der Endstufe 13 an. Dieser Ausgang ist über eine Leitung 13a mit einem Signaleingang des Mikrocontrollers verbunden, sodass im Mikrocontroller die tatsächlich an der Endstufe anliegende Signalstärke bestimmt werden kann. Der Mikrocontroller gibt nun über die Leitung 12a ein entsprechendes Signal an die Vorverstärkung 12, sodass am Ausgang der Endstufe 13 ein Signal mit der gewünschten Intensität anliegt. Zur Berechnung der gewünschten Intensität des am Ausgang der Endstufe 13 anliegenden elektrischen Signals werden von dem Mikrocontroller über die Leitung 14a die Kalibrierungsdaten des Ultraschallkopfes eingelesen. Mit Hilfe dieser Kalibrierungsdaten berechnet der Mikrocontroller die gewünschte Intensität am Ausgang der Endstufe 13 derart, dass durch den Ultraschallkopf Ultraschallstrahlung mit der vorgegebenen Intensität.von 1 W/cm² erzeugt wird.

Darüber hinaus ist der Mikrocontroller über eine Leitung 17b mit der Kopplungskontrolle 7 des Ultraschallkopfes 4 verbunden. Die Kopplungskontrolle gibt bei Anliegen des in Figur 1 dargestellten Protektors 3 an biologischem Gewebe ein Signal an den Mikrocontroller und der Mikrocontroller ist derart ausgeführt, dass er nur bei anliegendem Signal der Kopplungskontrolle ein positives Signal an die Vorverstärkung abgibt. Dies bedeutet, dass bei fehlendem Signal der Kopplungskontrolle, das heißt, wenn der in Figur 1 dargestellte Protektor 3 nicht an dem biologischen Gewebe anliegt, kein positives Signal an die Vorverstärkung abgegeben wird und somit keinerlei Verstärkung stattfindet, sodass am Ausgang der Endstufe 3 allenfalls ein Signal mit geringer Intensität anliegt.

## Patentansprüche

1. System zur Erzeugung von Ultraschallwellen, insbesondere zur kosmetischen und therapeutischen Behandlung mit einem elektrisch angesteuerten. Ultraschallkopf (14), der Ultraschallwellen mit zumindest zwei unterschiedlichen Frequenzen *f₁, f₂* erzeugt, wobei
eine Steuereinheit (16) permanent oder zumindest während vorgegebener Zeitspannen zwischen den zumindest zwei vorgegebenen Frequenzwerten *f₁, f₂* umschaltet,
**dadurch gekennzeichnet,**
**dass** das Umschalten mit einer Wechselfrequenz im Bereich von 10 Hz bis 10 000 Hz, insbesondere im Bereich von 50 Hz bis 5 000 Hz, vorzugsweise 100 Hz bis 2000 Hz erfolgt.

2. System nach Anspruch 1,
**dadurch gekennzeichnet,**
**dass** die Ultraschallwellen Frequenzen im Bereich von 20 kHz bis 20 MHz, insbesondere von 0,5 MHz bis etwa 10 MHz, vorzugsweise 0,7 MHz bis etwa 10 MHz aufweisen.

3. System nach Anspruch 1,
**dadurch gekennzeichnet,**
**dass** das Umschalten zwischen Ultraschallwellen erfolgt, deren Frequenzen etwa im Verhältnis 1:2 bis 1:10, insbesondere 1:2,5 bis 1:5, vorzugsweise etwa 1:3 zueinander stehen.

4. System zur Erzeugung von Ultraschallwellen nach Anspruch 1,
**dadurch gekennzeichnet,**
**dass** es einen Signalgenerator (11, 12, 13, 15) aufweist, der zumindest zwei Frequenzen *f₁, f₂* erzeugt, die in Ultraschallwellen mit entsprechenden Frequenzen umgewandelt werden.

5. System zur Erzeugung von Ultraschallwellen nach Anspruch 1 ,
**dadurch gekennzeichnet,**
**dass** der Signalgenerator einen Oszillator und eine Frequenzmodulations-Einheit umfasst, die aus der Oszillator-Frequenz zumindest zwei verschiedene Frequenzen erzeugt.

6. System zur Erzeugung von Ultraschallwellen nach Anspruch 4,
**dadurch gekennzeichnet,**
**dass** der Signalgenerator einen Oszillator (11), einen Frequenzcontroller (15) und eine Verstärkungseinheit (12, 13) umfasst,
wobei der Frequenzcontroller (15) mit dem Oszillator (11) verbunden und derart ausgeführt ist, dass er für eine vorgegebene Frequenz derart auf den Oszillator (11) einwirkt, dass dieser mit der vorgegebenen Frequenz schwingt und
wobei ein Ausgang des Oszillators (11) mit einem Eingang der Verstärkungseinheit (12, 13) verbunden und die Verstärkungseinheit derart ausgeführt ist, dass sie das am Eingang anliegende Signal in etwa um einen vorgegebnen Faktor verstärkt und
**dass** ein Ausgang der Verstärkungseinheit (12, 13) der Signalausgang des Signalgenerators ist.

7. System zur Erzeugung von Ultraschallwellen nach Anspruch 6,
**dadurch gekennzeichnet,**
**dass** die Steuereinheit (16) als Microcontroller mit einem Steuerausgang ausgeführt ist, welcher mit einem Eingang des Frequenzcontrollers (15) verbunden ist, zur Steuerung der durch den Signalgenerator erzeugten Frequenz.

8. System zur Erzeugung von Ultraschallwellen nach Anspruch 7,
**dadurch gekennzeichnet,**
**dass** die Steuereinheit (16) mit einem Steuereingang der Verstärkungseinheit (12, 13) verbunden ist,
**dass** die Steuereinheit (16) einen Signaleingang aufweist, welcher mit dem Signalausgang der Verstärkungseinheit (12, 13) verbunden ist und dass die Steuereinheit (16) derart ausgeführt ist, dass sie abhängig von der Signalstärke des am Signaleingang der Steuereinheit (16) anliegenden Signals derart über den Steuereingang der Verstärkungseinheit (12, 13) auf diese einwirkt, dass die Verstärkungseinheit (12, 13) ein periodisches elektrisches Signal mit einer durch die Steuereinheit (16) vorgegebenen Intensität erzeugt.

9. System zur Erzeugung von Ultraschallwellen nach einem der vorangegangenen Ansprüche,
**dadurch gekennzeichnet,**
**dass** das System mindestens zwei Ultraschallköpfe (14) insbesondere mit unterschiedlicher Größe aufweist und
**dass** der Signalausgang des Signalgenerators derart ausgeführt ist, dass wahlweise ein oder die mindestens zwei Ultraschallköpfe (14) gleichzeitig mit dem Signalausgang des Signalgenerators verbunden werden können.

10. System zur Erzeugung von Ultraschallwellen nach einem der vorangegangenen Ansprüche,
**dadurch gekennzeichnet,**
**dass** das System zusätzlich einen Reizstromgenerator aufweist, mit einem Reizstromausgang und
**dass** der Ultraschallkopf (14) eine Kontakteinheit zur Übertragung eines Reizstromes auf biologisches Gewebe aufweist und derart ausgeführt ist, dass die Kontakteinheit an den Reizstromausgang des Reizstromgenerators anschließbar ist.

11. System zur Erzeugung von Ultraschallwellen nach einem der vorangegangenen Ansprüche,
**dadurch gekennzeichnet,**
**dass** das System zusätzlich eine Vakuumeinheit aufweist mit einem Vakuumanschluss zum Erzeugen eines Unterdrucks und
**dass** der Ultraschallkopf (14) eine Vakuumglocke umfasst und derart ausgeführt ist, dass die Vakuumglocke an den Vakuumanschluss der Vakuumeinheit anschließbar ist, wobei der Ultraschallkopf und die Vakuumglocke derart ausgeführt sind, dass bei Anlegen des Ultraschallkopfes an biologisches Gewebe die Vakuumglocke nahezu fluiddicht an dem biologischen Gewebe anliegt, so dass zwischen biologischem Gewebe und Ultraschallkopf ein Unterdruck hergestellt werden kann.

12. System zur Erzeugung von Ultraschallwellen nach einem der vorangegangenen Ansprüche,
**dadurch gekennzeichnet,**
**dass** das System zusätzlich eine Infraroteinheit zur Erzeugung von Infrarotstrahlung aufweist, umfassend mindestens eine Infrarotstrahlungsquelle zur Erzeugung der Infrarotstrahlung und eine mit der Infrarotstrahlungsquelle verbundene Energieversorgung,
wobei die Infrarotstrahlungsquelie mit dem Ultraschallkopf (14) eine bauliche Einheit bildet.

13. System zur Erzeugung von Ultraschallwellen nach einem der vorangegangenen Ansprüche,
**dadurch gekennzeichnet,**
**dass** das System zusätzlich eine Vorrichtung zur Erzeugung von Hochfrequenzströmen mit einer Frequenz zwischen 300 kHz bis 10 MHz aufweist und dieser Strom an zumindest eine nach außen laufende Elektrode übertragen wird.

14. Verfahren zur Erzeugung von Ultraschallwellen insbesondere zur kosmetischen und therapeutischen Behandlung mit einem elektrisch angesteuerten Ultraschallkopf, der Ultraschallwellen mit zumindest zwei unterschiedlichen Frequenzen *f₁, f₂* erzeugt, wobei permanent oder zumindest zeitweise zwischen den zumindest zwei vorgegebenen Frequenzen *f₁, f₂* umgeschaltet wird,
**dadurch gekennzeichnet,**
**dass** das Umschalten mit einer Wechselfrequenz im Bereich von 10Hz bis 10 000 Hz, insbesondere im Bereich von 50 Hz bis 5 000 Hz, vorzugsweise von 100 Hz bis 2 000 Hz erfolgt.

15. Verfahren zum kosmetischen Fettbehandlung, insbesondere durch gezieltes Absaugen aus bestimmten Körperpartien,
**dadurch gekennzeichnet,**
**dass** die zu behandelnde Körperpartie vor und/oder nach dem Absaugen mit dem System gemäß einem der Ansprüche 1 bis 13 oder gemäß dem Verfahren nach Anspruch 14 behandelt wird.
